# EUROPEAN PATENT APPLICATION

(11) **EP 3 957 335 A1**
(43) Date of publication of application: **23.02.2022**
(21) Application number: 20191626.9
(22) Date of filing: 18.08.2020
(51) Int. Cl.: A61L 9/015

(54) **DEVICE FOR DIFFUSING VOLATILE SUBSTANCES**

(71) Applicant: Zobele Holding S.P.A., 38100 Trento (IT)
(72) Inventor: DEFLORIAN, Stefano, 38100 Trento (IT); MORHAIN, Cedric, 38100 Trento (IT)
(74) Representative: Herrero & Asociados, S.L.

(57) **Abstract**

The device for diffusing volatile substances comprises diffusion means (1) for diffusing the volatile substances; and control means (2) connected to the diffusion means (1) that control the operation of the diffusion means (1), wherein the device for diffusing volatile substances also comprises detection means (3) connected with the control means (2) for detecting if the device for diffusing volatile substances is worn by a user or is placed on a surface.

It permits to provide a device for diffusing volatile substances that can be used as a portable diffuser and also as a domestic diffuser, detecting automatically in which it is used and adapting its operation mode.

## Description

The present invention refers to a device for diffusing volatile substances.

### Background of the invention

Volatile substance diffusers have become a common object of everyday life, mostly for air-freshening or pest control purposes.

With the appearing of new technologies and also improvements in battery technologies, the application of portable solutions has become more and more frequent.

Today volatile substance diffusers can be portable for a coverage range quite limited to the surrounding of an individual.

Other volatile substance diffusers can be domestic or environmental, to cover larger spaces such as a room.

Nowadays, these different devices for diffusing volatile substances are designed completely for being used only for their intended use.

Therefore, a manufacturer that manufactures both kinds of diffusers has a double cost, one for each kind of diffuser.

In the market there is no device that is able to determine automatically in which situation it is used and adapts then its operation mode.

### Description of the invention

Therefore, one purpose of the present invention is to provide a device for diffusing volatile substances that can be used as a portable diffuser and also as a domestic diffuser, detecting automatically in which it is used and adapting its operation mode.

With the device for diffusing volatile substances according to the present invention it is possible to solve said drawbacks, providing other advantages that are described below.

The device for diffusing volatile substances comprises:
- diffusion means for diffusing the volatile substances; and
- control means connected to the diffusion means that control the operation of the diffusion means,
wherein the device for diffusing volatile substances also comprises detection means connected with the control means for detecting if the device for diffusing volatile substances is worn by a user or is placed on a surface.

According to possible embodiments, the detection means comprises a contact switch, a light sensor and/or a proximity sensor.

Preferably, the detection means is placed in the bottom part of the device.

Advantageously, the detection means sends a signal to the control means for setting the control means in a first operation mode when the device is worn by a user or in a second operation mode when the device is placed on a surface.

Also advantageously, in the first operation mode the length of the activation period of the diffusion means and/or the time between activation periods of the diffusion means is/are different from the second operation mode.

To prevent false detections, the detection means wait a verification period before sending the signal to the control means, such as e.g. 2-30 seconds.

### Brief description of the drawings

For a better understanding the above explanation and for the sole purpose of providing an example, some non-limiting drawings are included that schematically depict a practical embodiment.

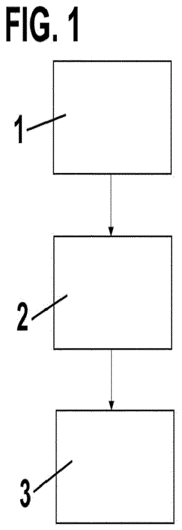

Figure 1 is block diagram of the components of the device for diffusing volatile substances according to the present invention; and

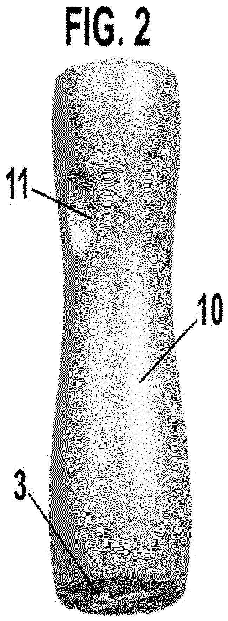

Figure 2 is a perspective of an embodiment of the device for diffusing volatile substances according to the present invention.

### Description of a preferred embodiment

The present invention refers to a device for diffusing volatile substances which is provided with active diffusion means 1, that can be controlled by electronic control means 2.

For example, the active diffusion means can be heating means, fan(s), nebulizer(s), piezoelectric atomizer(s), etc.

The device has different operation modes, defined in the present description as personal operation mode, when the device is worn by a person, and environmental operation mode, when the device is used in a room, i.e. placed on a surface such as a table.

Furthermore, the device for diffusing volatile substances according to the present invention also comprises means for detecting the operation mode 3, which determine which operation mode is used.

For example, in the case of an air freshener, it is clear that the usage to perfume the surrounding of a person will require a quiet lower output of evaporation rate regarding what will be needed to perfume a room.

Basically, the detection means 3 consists in means to detect the device is placed or not on a surface.

Such detection means 3 can be mechanical, such as a switch, that is pressed when the device is placed on a surface and unpressed when the device is hold by the user.

Alternatively, the detection means 3 are electronical means. For example, a light sensor, that detect light when the device is not placed on a surface and does not detect light when the bottom surface of the device is place in contact with a support surface.

Still alternatively, the detection means 3 can be a proximity sensor, with an IR light emitter and receptor, that are placed inside the device one next to another with a certain angle between them. When a surface is placed at a certain distance below the sensor, the light emitted is reflexed to the sensor. When there is no surface below the sensor or it is not at the right distance the reflected beam does not reach the sensor.

As an improvement, the detection is not made in a spontaneous manner, but on a longer period of time. This is to avoid that the device is briefly moved from the surface where it is placed, switches to personal mode, or even worse, if the device, being hold by the user, as the bottom accidentally covered for an instant and switch to high output environmental mode.

For this reason, a verification period is defined. This verification period starts when the device detects a change in its use that would require a switch to environmental or personal mode.

The switch is delayed till the end of the verification period. If after that period, the change of situation is confirmed through the detection means 3, then the switch of operation mode becomes effective. This verification period can have a duration from 2 to 30 seconds.

As an alternative to check at the beginning and at the end of the verification period, the system can also carry out several checks within verification period, to confirm the change of situation is continuous or not.

In a preferred embodiment, the diffusion means of the device is a nebulizer, consisting of a nebulization head 10 and a container 11.

For simplicity reasons, some details of the diffusion means of the device are not shown in the drawings because they are obvious for a skilled person and are of conventional type.

The container is made of a bottle with a capillary wick that extend from the bottom of the bottle, in contact with a fragrance, and extending up to the external part of the bottle.

At its top level, the wick is placed in contact with a perforated metallic membrane that is coupled with a piezoelectric actuator. This way, the wick is supplying the lower face of the metallic membrane with fragrance and upon electrical impulse to the piezoelectric actuator, the fragrance is expulsed in the atmosphere in the shape of a fine mist of droplets.

When the device is to be used in the personal mode, the impulse duration is set to a quarter of second with an emanation of 1 mg at each pulse. With a pulse every 15 minutes, we reach an emanation rate of 4 mg/h, that is enough to perfume the area around a person with a suitable intensity.

When the device is to be used in the environmental mode, to perfume a bigger area such as a room, the impulse is set to have a duration of 1 second, with an emanation of 4 mg per impulse. And with and impulse every 6 minutes, we reach an overall emanation rate of 40 mg/h, that is suitable for perfuming a room.

According to the shown embodiment, the device comprises on its bottom surface a contact switch 31. When the switch is pressed and maintained press (because the device is put on a surface), the device detects that must operate in the environmental mode and switch to the impulse mode as described above.

When switch 31 is not pressed, the device detects that it is not placed on a surface and then it is switched to the personal mode, as described above.

Even though reference has been made to a specific embodiment of the invention, it is obvious for a person skilled in the art that the device for diffusing volatile substances described herein is susceptible to numerous variations and modifications, and that all of the details mentioned can be substituted for other technically equivalent ones without departing from the scope of protection defined by the attached claims.

## Claims

1. Device for diffusing volatile substances, comprising:
- diffusion means (1) for diffusing the volatile substances; and
- control means (2) connected to the diffusion means (1) that control the operation of the diffusion means (1),
**characterized in that** the device for diffusing volatile substances also comprises detection means (3) connected with the control means (2) for detecting if the device for diffusing volatile substances is worn by a user or is placed on a surface.

2. Device for diffusing volatile substances according to claim 1, wherein the detection means (3) comprises a contact switch.

3. Device for diffusing volatile substances according to claim 1, wherein the detection means (3) comprises a light sensor.

4. Device for diffusing volatile substances according to claim 1, wherein the detection means (3) comprises a proximity sensor.

5. Device for diffusing volatile substances according to claim 1, wherein the detection means (3) is placed in the bottom part of the device.

6. Device for diffusing volatile substances according to claim 1, wherein the detection means (3) sends a signal to the control means (2) for setting the control means (2) in a first operation mode when the device is worn by a user or in a second operation mode when the device is placed on a surface.

7. Device for diffusing volatile substances according to claim 1 or 6, wherein in the first operation mode the length of the activation period of the diffusion means (1) and/or the time between activation periods of the diffusion means (1) is/are different from the second operation mode.

8. Device for diffusing volatile substances according to claim 6, wherein the detection means (1) wait a verification period before sending the signal to the control means (2).
